# EUROPEAN PATENT APPLICATION

(11) **EP 1 473 055 A2**
(43) Date of publication of application: **03.11.2004**
(21) Application number: 04018579.5
(22) Date of filing: 28.11.2000
(51) Int. Cl.: A61M 15/00

(54) **Inhalation delivery apparatus and method**

(30) Priority: 15.12.1999 GB 9929486
(62) Divisional of application: 00989900.6
(71) Applicant: GLAXO GROUP LIMITED, Greenford, Middlesex UB6 0NN (GB)
(72) Inventor: Legge, Coulton Heath Glaxo Wellcome plc., Hertfordshire SG12 ODP (GB); Mohammed, Hlack Glaxo Wellcome plc, Hertfordshire SG12 ODP (GB); Taylor, Anthony James Glaxo Wellcome plc, Hertforshire SG12 ODP (GB)
(74) Representative: Pike, Christopher Gerard

(57) **Abstract**

There is provided an inhaler apparatus (1) comprising:
(a) a heating chamber reservoir (R) for containing a propellant that is liquid at room temperature and standard atmospheric pressure;
(b) a heating mechanism (HE), said heating mechanism being operable for heating said liquid propellant to at least a predetermined temperature threshold in a sealed manner to increase pressure inside said heating chamber reservoir (R);
(c) an actuator (P) for opening said apparatus and releasing for inhalation by a human said heated propellant;
(d) a reservoir for containing said liquid propellant that is separate and distinct from said heating chamber reservoir (R); and
(e) in communication with said separate reservoir and with said heating chamber reservoir (R), a mechanism for metering said selected amount of said liquid propellant from said separate reservoir to said heating chamber reservoir (R),
wherein said heating chamber reservoir (R) receives and heats a selected amount of said liquid propellant in a sealed manner to increase pressure within said heating chamber reservoir (R).

## Description

### Technical Field

The present invention relates generally to inhalation devices and methods. More particularly, the present invention relates to an inhaler for delivery of a dose of inhaled dry powdered medicament or for a dose of a inhaled medicament from a solution or a suspension. There is described a method for delivering a selected dose of medicament from such a device for inhalation.

| Table of Abbreviations | |
|---|---|
| aq | aqueous |
| C | Centigrade |
| FPM | fluticasone propionate medicament |
| > | greater than |
| < | less than |
| µm | micrometer |
| µl | microliter |
| µg | microgram |
| mg | milligram |
| ml | milliliter |
| mm | millimeter |
| min | minute |
| psig | pounds per square inch gauge |
| SSM | salbutamol sulfate |
| | medicament |
| TIMP | twin impinger |
| w/v | weight/volume |

### Background Art

Asthma and other respiratory diseases are typically treated by the inhalation of an appropriate bioactive agent for deposition in the lungs in order to ease patient breathing and to increase air capacity. Various treatments are widely used for respiratory diseases, including dry powder inhalers, metered dose inhalers and nebulizers. A dry powder inhaler (DPI) allows inhalation of a powdered medicament (generally admixed with a powdered excipient). Metered dose inhalers and nebulizers permits delivery of a medicament from a solution or a suspension. MDIs meter a dose of the suspension or solution and use a low boiling point propellant to disperse the dose into particles through a spray nozzle. Nebulizers on the other hand rely on ultrasonic vibration to create atomizer mists of inhaled particles.

Various DPIs are shown in the prior art. Generally, these rely on a patient's breath or on a compressed volume of air or oxygen for delivery to aerosolize a dose of drug. Examples are well known in the art, as shown in U.S. Patent No. 3,921,637 to Bennie et al. (assignors to Bespak Industries Limited), U.S. Patent No. 4,841,964 to Hurka et al., U.S. Patent No. 5,503,869 to Van Oort (assigned to Glaxo Wellcome, Inc.), U.S. Patent No. 5,655,523 to Hodson et al. (assigned to Minnesota Mining and Manufacturing Company), and International Publication No. WO 94/20164 to Mulhauser et al. (assigned to Tenax Corporation). Aerosolization of dry powders may also be assisted by a gaseous propellant such as perfluoropentane, carbon dioxide, or other compressed gas as noted in U.S. Patent No. 5,503,869 to Van Oort; US 5,619,984 to Hodson (assigned to Astra Aktiebolag); and U.S. Patent No. 4,114,615 to Wetterlin (assigned to Aktiebolaget Draco).

MDIs for inhalation of a medicament solution or a medicament suspension, via using a gaseous propellant for delivery, are also well exemplified in the art. U.S. Patent No. 5,848,587 to King (assigned to Medi-Nuclear Corporation, Inc.) describes an apparatus, having a particular wafer closure means, for aerosol delivery of a medicament suspended in a chlorofluorocarbon gaseous propellant. Also, U.S. Patent No. 5,348,730 to Greenleaf et al. (assignors to Minnesota Mining and Manufacturing Company) describes a method for preparing a self-propelling, aerosol composition containing a finely divided medicament that is coated with a non-perfluorinated surface active dispersing agent and that is suspended in a gaseous propellant, the preferred propellant being 1,1,1,2-tetrafluoroethane.

Of note is the previously mentioned U.S. Patent No. 4,114,615 to Wetterlin, which describes an apparatus for aerosol delivery of a medicament by a chlorofluorocarbon gaseous propellant, with separate propellant and medicament containers, and useful for either dry powder medicament or liquid medicament.

US Patent No. 4,028,445 describes apparatus for wetting respiratory gas, in which a wetting chamber receives a heater cartridge. French Patent No. 834,229 describes apparatus for vaporizing medicament, in which dry powder medicament is comprised within a reservoir.

As is well known, a link has been established between gaseous chlorofluorocarbon emissions and the deterioration of the earth's atmospheric ozone layer. Hence, many attempts have been made to replace the previously employed chlorofluorocarbon propellants, for instance the well known chlorofluorocarbon propellant sold under the trademark FREON 12® by E.I. DuPont de Nemours and Company of Wilmington, Delaware. Specifically, FREON 12® is dichlorodifluoromethane and has an ozone depletion potential of 1. The propellant 1,1,1,2-tetrafluoroethane is sold under the trade name HFA 134a by ICI Chemicals and Polymers Limited of Runcorn, Cheshire, England. HFA 134a has a boiling point of -26.4°C and a vapor pressure at 20°C of 68.4 psig. As HFA 134a does not contain any chlorine moieties, HFA 134a is not a chlorofluorocarbon, and has been designated as a safe gaseous propellant having an ozone depletion potential of 0. However, more recent evidence has established that the ozone depletion potential of HFA 134a is not truly 0, but rather is extremely close to 0. Thus, on a large scale use, gaseous HFA 134a emissions can still result in deterioration of the earth's atmospheric ozone layer. Moreover, another drawback of HFA 134a is its high vapor pressure. Thus, HFA 134a must be used either (1) in conjunction with a vapor pressure depressant or (2) with a sturdy apparatus; otherwise, the apparatus may explode.

Accordingly, a replacement propellant that truly has an ozone depletion potential of 0 has long been sought for aerosol inhalers for delivery of a medicament solution or a medicament suspension, and such a propellant should also be useful for improved delivery by dry powder inhalers.

As is well known, solvents such as water or ethanol have an ozone depletion potential that is truly 0, but to the present inventor's knowledge, no one in the past has considered employing these solvents in an inhaler because these solvents are liquid at conditions of room temperature and standard atmospheric pressure, unlike HFA 134a and FREON 12®, which are vapors at these conditions.

Hence, the present inventor unexpectedly discovered that solvents such as water or ethanol, which are completely environmentally safe with an ozone depletion that is truly 0, could be employed in an inhaler for dispensing the medicament, as long as the solvent was heated for a very short time, i.e., a few seconds or less, in order to partially or completely vaporize the solvent so that the vapor propels the medicament for inhalation by the patient.

### Statement and Objects of the Invention

In accordance with the present invention, an inhalation delivery apparatus is provided for delivering medicament for inhalation. The inhaler apparatus comprises:
(a) a heating chamber reservoir for containing a propellant that is liquid at room temperature and standard atmospheric pressure;
(b) a heating mechanism, said heating mechanism being operable for heating said liquid propellant to at least a predetermined temperature threshold in a sealed manner to increase pressure inside said heating chamber reservoir;
(c) an actuator for opening said apparatus and releasing for inhalation by a human said heated propellant;
(d) a reservoir for containing said liquid propellant that is separate and distinct from said heating chamber reservoir; and
(e) in communication with said separate reservoir and with said heating chamber reservoir, a mechanism for metering said selected amount of said liquid propellant from said separate reservoir to said heating chamber reservoir,
wherein said heating chamber reservoir receives and heats a selected amount of said liquid propellant in a sealed manner to increase pressure within said heating chamber reservoir.

There is described a method for delivering a heated liquid from an apparatus. The method comprises heating a selected amount of a propellant that is liquid at room temperature and standard atmospheric pressure within a heating chamber reservoir of an apparatus to at least a predetermined temperature threshold in a sealed manner and increasing pressure within the heating chamber reservoir. Then, the method comprises releasing for inhalation by a human the heated propellant from the heating chamber reservoir of the apparatus such that the released heated propellant from the heating chamber reservoir propels through an exit orifice of the apparatus for inhalation.

Preferably, the liquid propellant is water. Hence, it is an object of the invention to provide an inhaler that may be used with an environmentally safe propellant, such as water vapor.

### Brief Description of the Drawings

Figure 1 of the drawings is an exploded perspective view of a first embodiment of the apparatus of the present invention, the first embodiment being suitable for use with medicament in liquid solution or liquid suspension;
Figure 2 of the drawings is a perspective view of the apparatus shown in Figure 1 but with components now connected as per when the apparatus is ready to be actuated, and also showing a mouth piece for delivery of released heated propellant to a patient's mouth;
Figure 3 of the drawings is substantially a cross-sectional view of the apparatus shown in Figure 1;
Figure 4 of the drawings is substantially a cross-sectional view of the apparatus shown in Figure 2, but not showing the mouth piece;
Figure 5 of the drawings is a plan view of an associated plug, voltage regulator, temperature sensor, and electrical connector for use with the apparatus of the invention in either the first or second embodiment;
Figure 6 of the drawings is an exploded perspective view of a second embodiment of the apparatus of the present invention, the second embodiment being suitable for use with dry powdered medicament;
Figure 7 of the drawings is a perspective view of the apparatus shown in Figure 6 but with components now connected as per when the apparatus is ready to be actuated, and also showing a mouth piece for delivery of released heated propellant to a patient's mouth;
Figure 8 of the drawings is substantially a cross-sectional view of the apparatus shown in Figure 6; and
Figure 9 of the drawings is substantially a cross-sectional view of the apparatus shown in Figure 7, but not showing the mouth piece.

### Detailed Description of the Invention

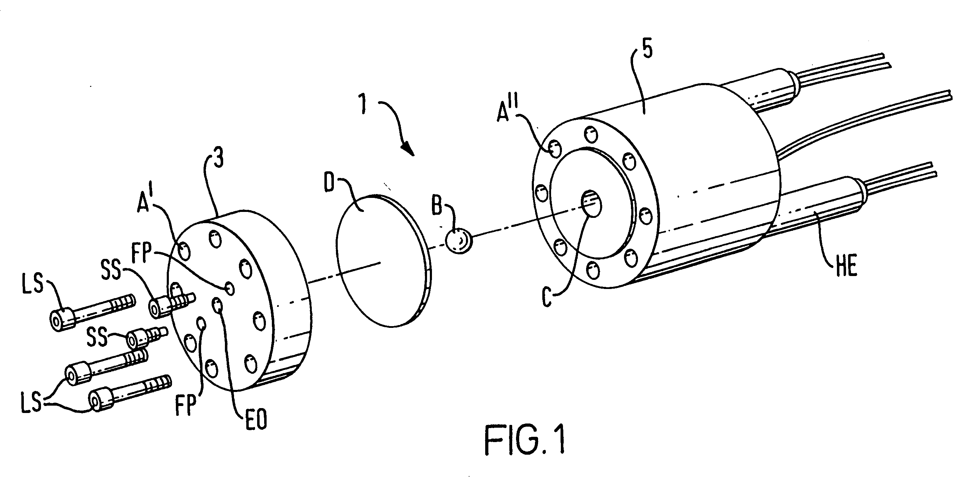

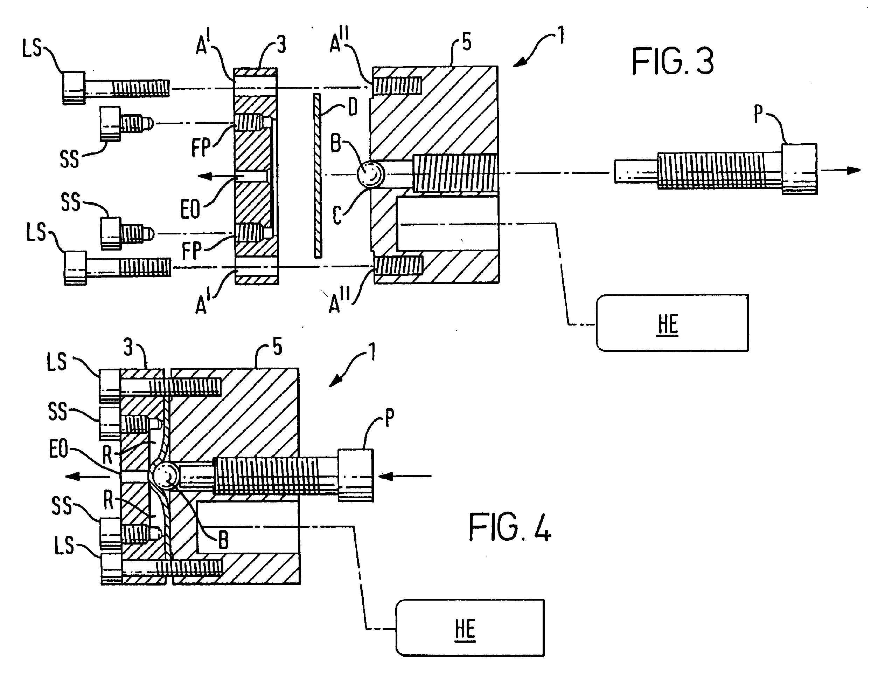

In accordance with the present invention, illustrated in Figure 1 is a perspective view, in exploded form, of apparatus **1** of the present invention in a first embodiment suitable for use as an inhaler with medicament in liquid solution or liquid suspension. The components of inhaler apparatus **1** may conveniently be made of stainless steel, and inhaler apparatus **1** may suitably be of a size to be a hand-held apparatus. More particularly, shown is front portion **3** and rear portion **5**, having between them diaphragm **D** and ball **B**. Front portion **3** contains a plurality of apertures **A'**, whereas rear portion **5** contains a plurality of corresponding apertures **A**", for receiving connecting means, shown as long screws **LS**, for connecting together front portion **3** and rear portion **5**. It is noted that for the embodiment shown, there would be eight long screws **LS** for each of the eight pairs of apertures **A**', **A**", but for purposes of clarity, only three long screws **LS** are shown in Figure 1. Also, front portion **3** has at least one filling port **FP** for placing a propellant (not shown in Figure 1) that is liquid at room temperature and standard atmospheric pressure into front portion **3**. Heating element **HE** (partially shown in Figure 1, but see Figure **3** for a better view of how heating element **HE** is received in rear portion **5**) is provided for heating the liquid propellant.

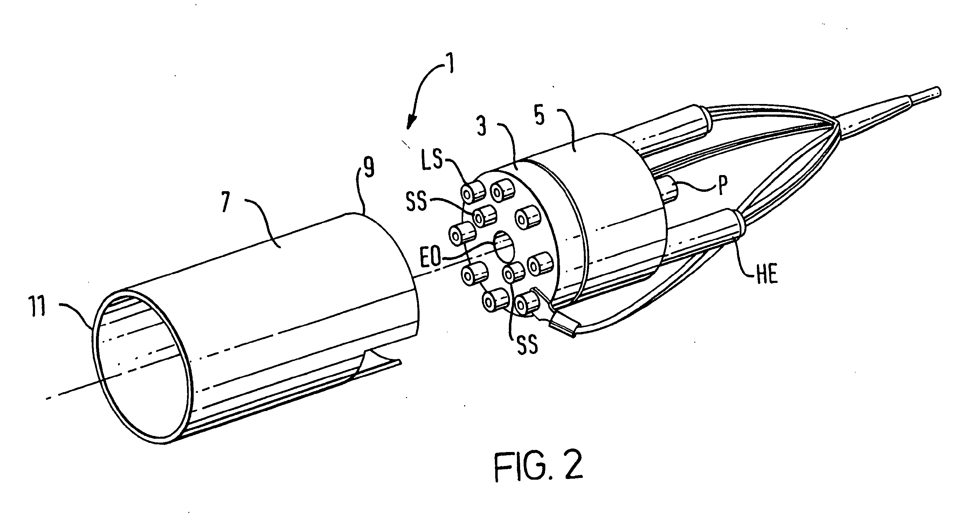

With reference now to Figure 2, each pair of aperture **A**' and corresponding aperture **A**" is shown containing a connector means, illustrated as long screw **LS**, connecting front portion **3** and rear portion **5** with diaphragm **D** and ball **B** between them. (Diaphragm **D** and ball **B** cannot be seen in Figure 2.) As discussed in more detail below, when the actuator, illustrated as a manual release, for instance screw-in pin **P**, is actuated, heated propellant is released via exit orifice **EO** and through spacer or mouth piece **7** (illustrated as a hollow tube) that suitably has back end **9** for sliding onto front portion **3** and front end **11** for placing in the mouth of a patient (not shown). It is noted that a mouth piece is preferred, but not required. It is further noted that manual trigger actuators, besides manual pin actuators, are also well known, as are actuators that are temperature actuated, actuators that are pressure actuated, and actuators that are breath actuated, all being suitable for use in this first embodiment or in the below-described second embodiment.

Figure 3 is substantially a cross-sectional view of inhaler apparatus **1** of Figure 1. Figure 4 is substantially a cross-sectional view of inhaler apparatus **1** of Figure 2, but not showing spacer or mouth piece **7**. As can be seen from Figures 3 and 4, filling port **FP** communicates with reservoir **R**, which in turn communicates with exit orifice **EO**, for release of heated propellant to the patient's mouth. Reservoir **R** serves as a heating chamber as further discussed below.

More specifically, in order to fill inhaler apparatus **1** with the liquid propellant, front portion **3** and rear portion **5** are connected by tightening long screws **LS** into each pair of associated apertures **A', A"** and tightening pin **P** in channel **C** so that pin **P** pushes against ball **B**, which in turn, pushes against diaphragm **D**, which flexes slightly in order to seal off each reservoir **R**. The liquid propellant is then placed into each filling port **FP**, conveniently by use of a syringe (not shown), and travels from each filling port **FP** to each reservoir **R**. Pin **P** may be slightly loosened in channel **C**, which will result in diaphragm **D** moving slightly away from exit orifice **EO** and thus some liquid propellant pouring out of exit orifice **EO**. That way, it will be known when each reservoir **R** is full. Then, previously slightly loosened pin **P** is now tightened in channel **C** in order to force ball **B** against diaphragm **D**, which flexes slightly and seals closed exit orifice **EO**, and also short screws **SS** are inserted into filling ports **FP** to close them. Consequently, the liquid propellant is now trapped in each sealed closed reservoir **R**.

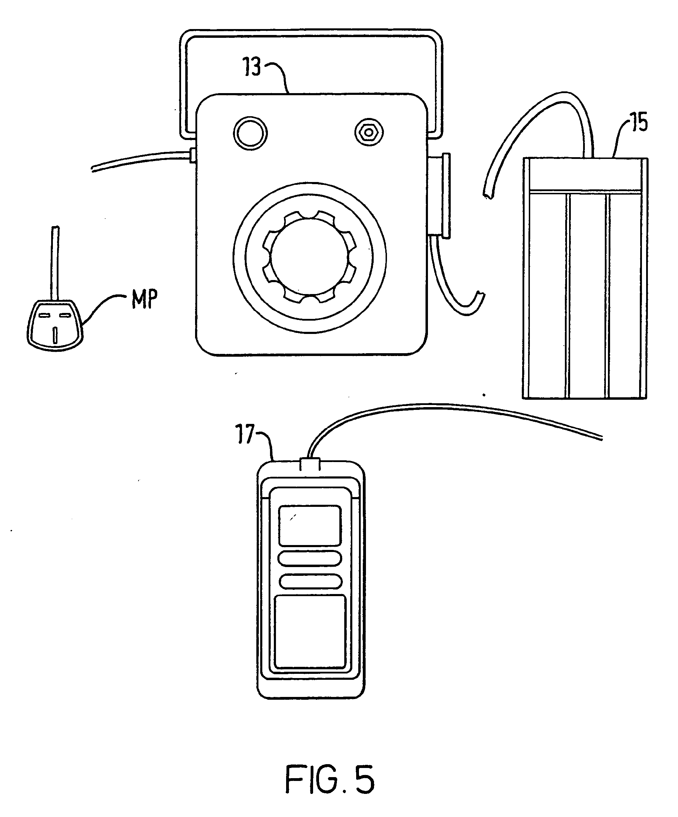

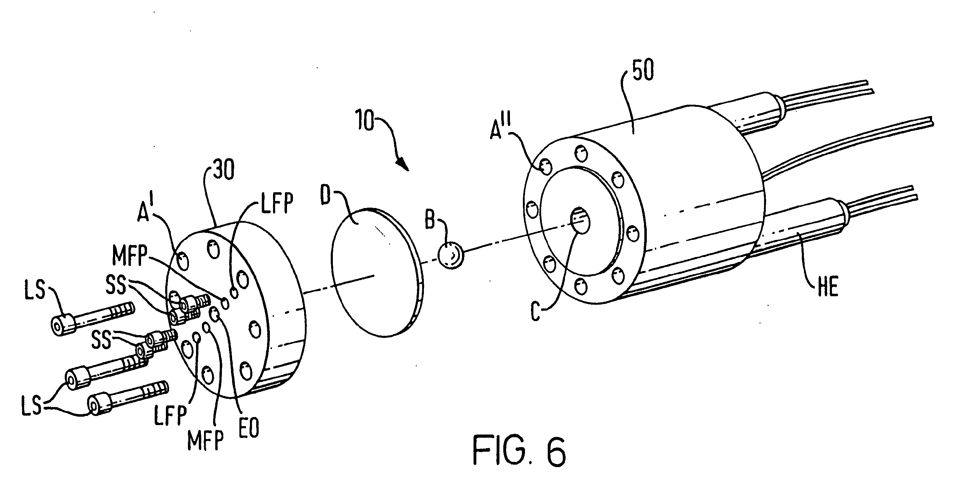

As shown in Figure 5, provided also are associated voltage regulator **13**, electrical connector **15**, and temperature sensor **17**, which may be suitably connected to an electrical source via male plug **MP** as shown, or may be battery operated (not shown) in order to provide electrical connection and thus heat up the liquid propellant, sealed inside reservoir **R**, via heating element **HE** (see Figures 1, 2, 3, and 4). Thus, reservoir **R** also serves as a heating chamber. The liquid propellant in inhaler apparatus **1** is then heated to a desired temperature (which will depend on which liquid propellant is employed), with the temperature being monitored by way of temperature sensor **17**. As the temperature increases, the pressure increases, and upon release, the liquid propellant will partially or completely vaporize. (The same voltage regulator **13**, electrical connector **15**, temperature sensor **17**, and male plug **MP** are also suitable for use with the alternative embodiment described below with respect to Figures 6, 7, 8, and 9.)

While it is not intended to be bound to any theory, it is not known, because the heating chamber is a sealed closed system, whether the pressure increase from the heating causes the liquid propellant to vaporize inside the heating chamber or causes the liquid propellant to remain as a super-heated liquid inside the heating chamber. Of course, this can also depend on the particular liquid propellant employed. However, when the actuator opens the apparatus, then heated propellant is released for inhalation. Thus, already vaporized propellant may be released; super-heated propellant may vaporize immediately after release; and/or liquid droplets may co-exist with a gaseous phase, or a combination thereof. Hence, the term "vapor" is used herein to refer to a state where a the dose is in a gaseous state, a superheated liquid state to form gaseous and/or liquid droplet components on release, or a as a combination of gaseous and liquid droplets upon release.

Suitable liquid propellants (liquid at room temperature and standard atmospheric pressure) include, but are not limited to, water, alkanols (such as ethanol), glycols (such as ethylene glycol, propylene glycol, or polyethylene glycol), ethanolamine, oleic acid, anti-oxidants (such as butylated hydroxytoluene or butylated hydroxyanisole), lecithin, surfactants (such as sorbitan trioleate), and the like. Preferably, the liquid propellant should be heated to at least about 30°C above up to at least about 80°C above, and more preferably at least about 40°C above, its boiling point in °C. Also, preferably from the heating, the liquid propellant should be pressurized to a pressure increase greater than atmospheric pressure, i.e., a net positive pressure increase, in order to force the propellant out of the heating chamber. For this first embodiment vis-a-vis Figures 1, 2, 3, and 4, the propellant contains one or more of various medicaments suitable for inhalation therapy, such as FPM, SSM, and the like, dispersed in the propellant.

Heating is initiated, and when the desired temperature (or the desired pressure) is achieved, inhaler apparatus **1** is placed with front end **11** of spacer or mouth piece **7** in the patient's mouth, followed by releasing pin **P**. Release of pin **P** causes loosening of ball **B** from its position up against diaphragm **D** (see Figure 4) so that diaphragm **D** moves away from front portion **3** (see Figure 3) and the heated propellant comes out in the direction of the arrow shown in exit orifice **EO** (see Figure 3), traveling through spacer or mouth piece **7** and into the patient's mouth, for inhalation and deposition of the medicament into the patient's lungs. It is noted that Figure 3 is exploded for clarity, and that actually movement will be very slight of diaphragm **D** away from front portion **3** for dispensing heated propellant.

Various sizes, suitably ranging from an internal diameter of 0.1 mm to 0.7 mm, may be employed for exit orifice **EO**. Also, as described in Example 6 below, a parallel sided projection, which may be made of brass, optionally may be attached post exit orifice **EO** to decrease the angle of the spray of the vapor, and a suitable internal diameter for such a parallel, sided projection is 0.6 mm.

With reference now to Figure 6, shown is a perspective view, in exploded form, of a second embodiment of the present invention indicated as inhaler apparatus **10** and suitable for use with dry powdered medicament. The components of inhaler apparatus **10** may conveniently be made of stainless steel. Inhaler apparatus **10** is essentially like inhaler apparatus **1**, except for having separate and distinct filling ports and reservoirs for the dry powder medicament and the liquid propellant, respectively, and thus, inhaler apparatus **10** may suitably be of a size to be a hand-held apparatus.

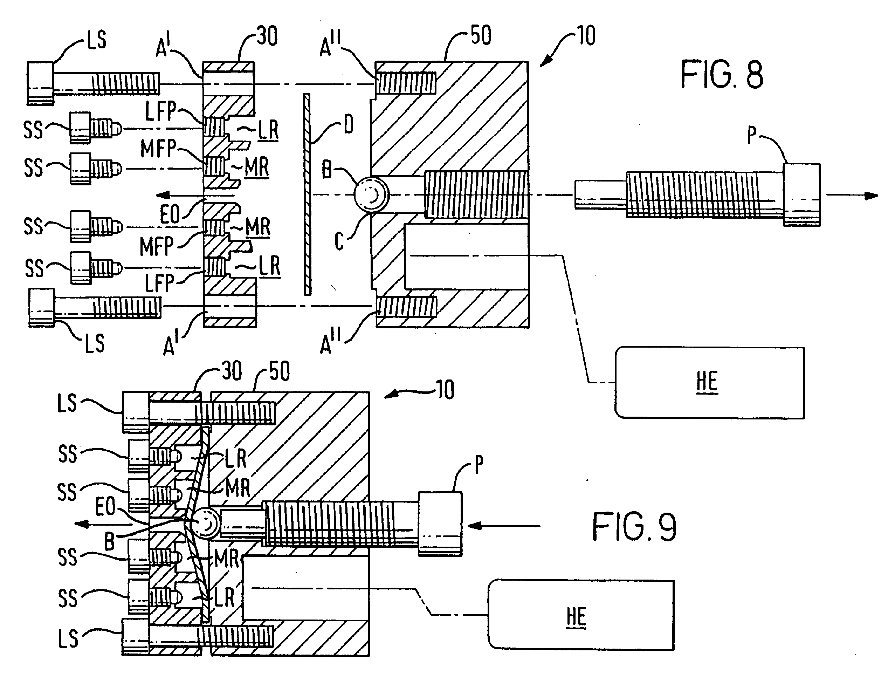

More particularly, shown is front portion **30** and rear portion **50**, having between them diaphragm **D** and ball **B**. Front portion **30** contains a plurality of apertures **A'**, whereas rear portion **50** contains a plurality of corresponding apertures **A"**, for receiving connecting means, shown as long screws LS, for connecting together front portion **30** and rear portion **50**. It is noted that for the embodiment shown, there would be eight long screws **LS** for each of the eight pairs of apertures **A'**, **A"**, but for purposes of clarity, only three long screws **LS** are shown in Figure 6. Also, front portion **30** has at least one liquid filling port **LFP** for placing a propellant (not shown in Figure 6) that is liquid at room temperature and standard atmospheric pressure into front portion **30**, and front portion **30** has at least one separate and distinct medicament filling port **MFP** for placing dry powdered medicament. Heating element **HE** (partially shown in Figure 6, but see Figure 8 for a better view of how heating element **HE** is received in rear portion **50**) is provided for heating the liquid propellant.

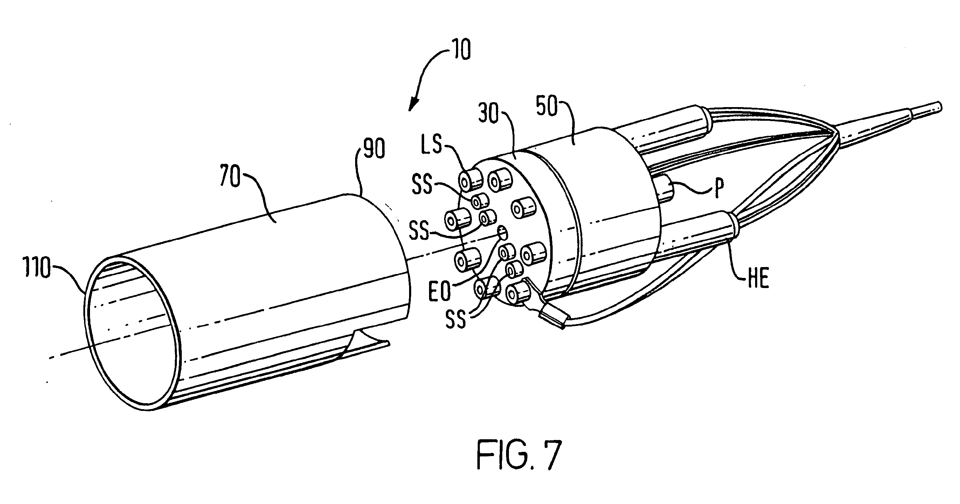

With reference now to Figure 7, each pair of aperture **A'** and corresponding aperture **A"** is shown containing a connector means, illustrated as long screw **LS**, connecting front portion **30** and rear portion **50** with diaphragm **D** and ball **B** between them. (Diaphragm **D** and ball **B** cannot be seen in Figure 7.) As discussed in more detail below, when the actuator, illustrated as pin **P**, is actuated, heated propellant is released via exit orifice **EO** and through spacer or mouth piece **70** (illustrated as a hollow tube) that suitably has back end **90** for sliding onto front portion **30** and front end **110** for placing in the mouth of a patient (not shown). As noted above vis-a-vis inhaler apparatus **1**, likewise a mouth piece is not required and various other kinds of actuators may be employed with inhaler apparatus **10**.

Figure 8 is substantially a cross-sectional view of inhaler apparatus **10** of Figure 6. Figure 9 is substantially a cross-sectional view of inhaler apparatus **10** of Figure 7, but not showing spacer or mouth piece **70**. As can be seen from Figures 8 and 9, liquid filling port **LFP** communicates with its liquid reservoir **LR** and medicament filling port **MFP** communicates with its medicament reservoir **MR**. In turn when inhaler apparatus **10** is actuated, reservoirs **LR** and **MR** communicate with each other and also communicate with exit orifice **EO**, for release of heated propellant to the patient's mouth. As can be seen from Figures 8 and 9, with respect to the positioning of liquid reservoir **LR** and medicament reservoir **MR** in relation to each other, liquid reservoir **LR** is more proximate to the outside of the apparatus whereas medicament reservoir **MR** is more proximate to the exit orifice, and likewise, with respect to the positioning of liquid filling port **LFP** and medicament filling port **MFP** in relation to each other, liquid filling port **LFP** is more proximate to the outside of the apparatus whereas medicament filling port **MFP** is more proximate to the exit orifice.

More specifically, in order to fill inhaler apparatus **10** with liquid propellant and dry powdered medicament, front portion **30** and rear portion **50** are connected by tightening long screws **LS** into each pair of associated apertures **A'**, **A"** and tightening pin **P** in channel **C** so that pin **P** pushes against ball **B**, which in turn, pushes against diaphragm **D**, which flexes slightly in order to seal off each reservoir **LR** and **MR**.

The liquid propellant is then placed into each liquid filling port **LFP**, conveniently by use of a syringe (not shown), and travels from each liquid filling port **LFP** to each liquid reservoir **LR**. Pin **P** may be slightly loosened in channel **C**, which will result in diaphragm **D** moving slightly away from exit orifice **EO** and thus some liquid propellant pouring out of exit orifice **EO**. That way, it will be known when each liquid reservoir **LR** is full. Then, previously slightly loosened pin **P** is now tightened in channel **C** in order to force ball **B** against diaphragm **D**, which flexes slightly and seals closed exit orifice **EO**, and also short screws **SS** are inserted into liquid filling ports **LFP** to close them. Consequently, the liquid propellant is now trapped in each liquid reservoir **LR**.

Then, dry powdered medicament is placed in each medicament reservoir **MR** by way of each medicament filling port **MFP**, and short screws **SS** are inserted into medicament filling ports **MFP** to close them and seal off each medicament reservoir **MR**.

Suitable liquid propellants are the same as described above with respect to the first embodiment. Suitable medicaments are also the same as long as they are dry powder, and not liquid.

Heating is initiated, and thus, liquid reservoir **LR** also serves as a heating chamber. The liquid propellant in inhaler apparatus **10** is then heated to a desired temperature or a desired pressure (which will depend on which liquid propellant is employed), with the temperature being monitored by way of temperature sensor **17**. As the temperature of the liquid propellant increases, the pressure of the liquid propellant increases.

When the desired temperature (or pressure) is achieved, inhaler apparatus **10** is placed with front end **110** of spacer or mouth piece **70** in the patient's mouth, followed by releasing pin **P**. Release of pin **P** causes loosening of ball **B** from its position up against diaphragm **D** (see Figure 9) so that diaphragm **D** moves away from front portion **30** (see Figure 8). Then, in the direction of the arrow shown in exit orifice **EO** (see Figure 8), the heated propellant comes out, sweeping with it the dry powder medicament as they travel through spacer or mouth piece **70** and into the patient's mouth, for inhalation and deposition of the dry powder medicament into the patient's lungs. It is noted that Figure 8 is exploded for clarity, and that actually movement will be very slight of diaphragm **D** away from front portion **30** for dispensing heated propellant. While it is not intended to be bound to any theory, it is believed that the released propellant passes as a vapor (which includes vapor, liquid droplets, superheated liquid, or combinations thereof) through the dry powder medicament to propel the dry powder medicament or the released propellant passes by as a vapor and picks up the dry powder medicament to propel the dry powder medicament.

As described above in connection with the first embodiment, likewise for the second embodiment, various sizes, suitably ranging from an internal diameter of 0.1 mm to 0.7 mm, may be employed for exit orifice **EO**. Also, a parallel sided projection, which may be made of brass, optionally may be attached post exit orifice **EO** to decrease the angle of the spray of the vapor, and a suitable internal diameter for such a parallel sided projection is 0.6 mm.

It is noted that metering mechanisms for metering a selected dose of medicament for release by an inhaler apparatus to a patient are well known, and thus, either embodiment of inhaler apparatus **1** or **10** may be easily adapted, by the person of ordinary skill in the art without undue experimentation, to include a metering mechanism.

In one version of a metering mechanism, inhaler apparatus **1** would contain a plurality of reservoirs **R**, each containing one dose of medicament (dispersed in the liquid propellant in solution or suspension), the plurality may be individual blisters, or joined blisters, being distributed, for example along an elongate strip, or rotating disc near the disc's circumference. Similarly, Inhaler apparatus **10** would contain a plurality of medicament reservoirs **MR**, each containing one dose of dry powder, and a plurality of liquid reservoirs **LR**, each containing one dose of liquid propellant, the plurality may be individual blisters, or joined blisters, being distributed, for example along an elongate strip, or on a rotating disc near the disc's circumference. To operate inhaler **1**, one reservoir **R** with one dose of medicament would be sequentially aligned for communication with exit orifice **EO**. Likewise, to operate inhaler **10**, one medicament reservoir **MR** and one associated liquid reservoir **LR** would be aligned for communication with each and for in communication with exit orifice **EO**. The aligned liquid portion would of either inhaler **1** or **10** would then be heated/pressurized and released when the inhaler is actuated, for example by actuation of pin **P**, to release an individual dose of medicament to the patient. Subsequent alignment of the next reservoir could then be accomplished by insertion of the next unit dose, advancing the strip, rotating a disk to place the next reservoir with one dose of medicament in position for release during actuation.

It is noted that such unit dose, blister discs, and elongate strip presentations for use in DPI and MDI inhalers are well known, with the reservoirs containing the respective individual doses being colloquially referred to as "capsules," "sealed blisters" etc.., and thus, the inventive inhaler apparatus may be easily adapted for use therewith, without undue experimentation.

Additionally, metering is well known where liquid is in a separate and distinct reservoir, and then a selected amount is metered or pumped, such as via a communicating tube, from the reservoir to an inhaler apparatus. In other words, in the present invention, a selected amount of liquid propellant is pumped from the separate reservoir to the heating chamber. In this version, medicament could be dispersed in the liquid propellant that is contained in the separate and distinct reservoir, whereby one dose of the liquid propellant containing one dose of medicament would be pumped, for instance, to reservoir **R** as shown in Figure 4, or the separate and distinct reservoir would contain only liquid propellant, whereby one dose of liquid propellant would be pumped, for instance, as shown in Figure 8, to liquid reservoir **LR** while dry powder medicament would be already present in associated medicament reservoir **MR**. Such separate and distinct reservoirs for containing the liquid propellant, and associated pumping or metering mechanisms, are well known in the art, and thus, the inventive inhaler apparatus may be easily adapted for use therewith, without undue experimentation.

### EXAMPLES

The first embodiment of the inventive inhaler device was tested, using the drugs, fluticasone propionate (FPM) and salbutamol sulfate (SSM), with each dispersed in a separate aliquot of deionized water as the liquid propellant, by releasing the pin of the inhaler device to dispense vapor into a twin impinger (TIMP) analyzer attached to the exit orifice end of the inhaler device. The results are set out below in Examples 1-6, and it is expected that such tests on the second embodiment of the inventive inhaler device would demonstrate similar favorable results.

More particularly, the TIMP is a piece of glassware that allows air to be drawn through it at about 60 liters/min, the average inhalation rate for a human. Vapor was sprayed into the TIMP, which mimics the human airway system, and subsequently, the TIMP was taken apart into 2 halves referred to below as Stages 1 and 2. Drug was rinsed off each Stage; each of the two respective solutions (or suspensions) of the water containing the drug was then analyzed for average particle size and weight. Stage 1 contained particles with an average size > about 5.8 pm and Stage 2 contained particles with an average size < about 5.8 µm. Stage 2 contained the fraction of drug that would give a therapeutic response for deposition in a patient's lungs. Therefore, it is desirable to increase deposition in Stage 2.

The inhaler device was also tested, with its exit orifice end attached to a spacer, and with the TIMP attached at the other end of the spacer to capture the vapor, by then releasing the pin of the inhaler device to dispense vapor via the spacer into the TIMP. The spacer is essentially a hard oval shaped cylinder with two openings, one to attach to the inhaler device and the other for the patient to inhale through. As the patient inhales through the spacer, it allows the larger particles from a vapor to be deposited within it and not the patient. Therefore, the patient inhales mainly the smaller particles (average particle size < about 5.8 microns) which would give a therapeutic effect and not the larger ones (average particle size > about 5.8 microns) that are of no significant advantage. The reason for the use of a spacer in the tests below was to add a greater distance between the inhaler device and the entrance to the TIMP so that the vapor would slow down before entering the TIMP, allowing for defining the difference in droplet deposition with respect to droplet size and/or velocity. In other words, the slow down allowed for a determination as to whether the velocity of the vapor was so high that deposition would occur in Stage 1 irrespective of droplet size.

The following is noted with regard to the aqueous formulation of each drug. FPM nebules were an aqueous suspension containing:

| | |
|---|---|
| FPM | 1 mg/ml |
| polysorbate (surfactant) | 0.08 mg/ml |
| sorbitan monolaurate (surfactant) | 0.01 mg/ml |
| monosodium phosphate, dehydrate | 9.4 mg/ml |
| dibasic sodium phosphate, anhydrous | 1.75 mg/ml |
| sodium chloride | 4.8 mg/ml |

The ingredients in the FPM formulation in addition to FPM were to allow a good aqueous suspension of drug to be formed, i.e., drug particles were homogeneously dispersed in the formulation when shaken. The SSM aqueous solution contained SSM in an amount of 7.5 mg/ml and NaCl in an amount of 0.9% w/v. The NaCl was present in each formulation to make it isotonic with human body fluids, as would be the situation for absorption by the lungs when actually inhaled by a human patient.

As can be seen from the results below as per Examples 1-6, the inventive device deposited suitable amounts of drug.

### Example 1

### Test conditions:

Sample: FPM nebules, 1 mg/ml
Temp: 115°C
Dose volume: 100 µl
Exit orifice diameter of device: 0.70 mm

### Device attached to spacer A and to the TIMP:

| | Spacer Deposition | | Stage 1 Deposition | | Stage 2 Deposition | | Total Drug Deposited |
|---|---|---|---|---|---|---|---|
| Sample | µg | % | µg | % | µg | % | µg |
| 1 | 161.51 | 100 | 0.00 | 0 | 0.00 | 0 | 161.51 |
| 2 | 388.23 | 100 | 0.00 | 0 | 0.00 | 0 | 388.23 |

### Device attached to spacer B and to the TIMP:

| | Spacer Deposition | | Stage 1 Deposition | | Stage 2 Deposition | | Total Drug Deposited |
|---|---|---|---|---|---|---|---|
| Sample | µg | % | µg | % | µg | % | µg |
| 1 | 139.68 | 42 | 190.92 | 57 | 2.67 | 1 | 333.27 |

### Example 2

### Test conditions:

Sample: FPM nebules, 1 mg/ml
Temp: 140°C
Dose volume: 100 µl
Exit orifice diameter of device: 0.35 mm

### Device attached to spacer A and to the TIMP:

| | Spacer Deposition | | Stage 1 Deposition | | Stage 2 Deposition | | Total Drug Deposited |
|---|---|---|---|---|---|---|---|
| Sample | µg | % | µg | % | µg | % | µg |
| 1 | 237.88 | 88 | 4.02 | 1 | 29.40 | 11 | 271.30 |
| 2 | 275.09 | 88 | 5.32 | 2 | 31.12 | 10 | 311.53 |

### Example 3

### Test conditions:

Sample: FPM nebules, 1 mg/ml
Temp: 140°C
Dose volume: 30 µl
Exit orifice diameter of device: 0.35 mm

### Device attached to spacer A and to the TIMP:

| | Spacer Deposition | | Stage 1 Deposition | | Stage 2 Deposition | | Total Drug Deposited |
|---|---|---|---|---|---|---|---|
| Sample | µg | % | µg | % | µg | % | µg |
| 1 | 60.14 | 81 | 1.45 | 2 | 12.73 | 17 | 74.32 |
| 2 | 30.24 | 74 | 2.07 | 5 | 8.70 | 21 | 41.01 |

### Device attached to the TIMP (no spacer):

| | Stage 1 Deposition | | Stage 2 Deposition | | Total Drug Deposited |
|---|---|---|---|---|---|
| Sample | µg | % | µg | % | µg |
| 1 | 39.97 | 81 | 9.46 | 19 | 49.43 |
| 2 | 35.49 | 82 | 7.83 | 18 | 43.32 |

### Example 4

### Test conditions:

Sample: FPM nebules, 1 mg/ml
Temp: 140°C
Dose volume: 30 µl

### Device attached to the TIMP (no spacer):

| | Stage 1 Deposition | | Stage 2 Deposition | | Total Drug Deposited |
|---|---|---|---|---|---|
| Exit Orifice diameter of device | µg | % | µg | % | µg |
| 0.1 mm | 39.58 | 96 | 1.44 | 4 | 41.02 |
| 0.2 mm | 61.05 | 95 | 3.07 | 5 | 64.12 |
| 0.3 mm | 50.53 | 93 | 3.76 | 7 | 54.29 |

### Test conditions:

Sample: SSM aq. solution, 7.5 mg/ml
Temp: 140°C
Dose volume: 30 µl

### Device attached to the TIMP (no spacer):

| | Stage 1 Deposition | | Stage 2 Deposition | | Total Drug Deposited |
|---|---|---|---|---|---|
| Exit Orifice Diameter of device | µg | % | µg | % | µg |
| 0.1 mm | 777.0 | 94 | 49.7 | 6 | 826.7 |
| 0.2 mm | 697.1 | 96 | 29.2 | 4 | 726.3 |
| 0.3 mm | 635.3 | 95 | 32.0 | 5 | 667.3 |

### Example 5

### Test conditions:

Sample: FPM nebules, 1 mg/ml
Exit orifice diameter of device: 0.2 mm
Dose volume: 30 µl

### Device attached to the TIMP (no spacer):

| Sample | Stage 1 Deposition | | Stage 2 Deposition | | Total Drug Deposited |
|---|---|---|---|---|---|
| Temp. | µg | % | µg | % | µg |
| 130°C | 134.88 | 95 | 6.43 | 5 | 141.31 |
| 160°C | 109.57 | 87 | 15.74 | 13 | 125.31 |
| 180°C | 94.35 | 85 | 22.03 | 15 | 143.43 |

### Device attached to spacer A and to the TIMP:

| Sample | Spacer Deposition | | Stage 1 Deposition | | Stage 2 Deposition | | Total Drug Deposited |
|---|---|---|---|---|---|---|---|
| Temp. | µg | % | µg | % | µg | % | µg |
| 180°C | 94.34 | 72 | 5.10 | 4 | 32.00 | 24 | 131.45 |

### Example 6

### Test conditions:

Brass projection affixed post exit orifice: parallel sided, 0.6 mm internal diameter
Sample: FPM nebules, 1 mg/ml
Temp: 180°C
Dose volume: 30 µl
(only 4 doses collected)

### Device attached to the TIMP (no spacer):

| | Stage 1 Deposition | | Stage 2 Deposition | | Total Drug Deposited |
|---|---|---|---|---|---|
| Exit Orifice diameter of device | µg | % | µg | % | µg |
| 0.1 mm | 55.70 | 82 | 11.97 | 18 | 67.67 |
| 0.2 mm | 98.66 | 77 | 28.74 | 23 | 127.40 |
| 0.3 mm | 86.91 | 86 | 13.89 | 14 | 100.80 |

### Alternative embodiments

As will be appreciated by those of ordinary skill in the art, the inhaler of the present invention can be constructed of any suitable material or combination of materials, including, but not limited to metals, such as stainless steel, aluminum, and nickel; plastics; ceramics; and polymers. Likewise, the chambers, reservoirs, passageways and other features within or on the device may be coated or treated to improve their performance properties with any suitable materials. For example, passageways within the device may be coated with a polymeric materials or blends thereof to reduce any tendency for particle deposition. The polymeric materials include polyethyhlene (PE), polypropylene (PP), polymethylmethylacrylate (PMMA), polyvinyl chloride (PVC), polyvinyldiene chloride (PVDC), polyvinyl fluoride (PVF), polyvinyldiene fluoride (PVDF), polychlorotrifluoroethylene (PCTFE), polytetrafluoroethylene (PTFE), fluorinated ethylene propylene (FEP), perfluroroalkoxy (PFA), polyamide (PA), polyethylene terephthalate (PET), polybutylene terephthalate (PBT), polyetherimide (PEI), polyamideimide (PAI), polyimide (PI), polysulfone (PS), polyarylsulfone (PAS) polyethersulfone (PES), polyphenylene sulfide (PPS), polyetheretherketone (PEEK), polydimethylsiloxane (PDMS) and polycarbonate (PC) or combinations and blends thereof.

These polymeric materials are available from typical suppliers such as DuPont, Dow, General Electric, ICI, 3M, Monsanto, Amoco, BASF, Allied Signal, Bayer, Eastman, Phillips, LNP etc.

Other coatings may be employed to provide strengthen, insulate, and/or provide conductive characteristics.

The heat generating component of the device can be any device or material capable of creating sufficient thermal energy, and may include, without limitation, mechanical or kinetic heating elements; electromechanical heating elements; electrochemical heating elements; electromagnetic wave heating elements;; combustion based heating elements; chemical and physico/chemical heating elements.

Mechanical / kinetic heating elements include, without limitation, frictional heat generated by a wheel or physical component rubbing on a stationery component. Electrochemical heating elements include without limitation: AC or DC power source (either generator or storage unit, including power lines, battery, and/or solar cells) driven heaters such as heating rods, coils, resistive coating and/or linings; and heat pumps, or AC or DC driven cooling devices where cooling one portion of the system heats another portion of the system. Electromagnetic wave heating elements include, without limitation, microwaves, high intensity light, and lasers. Combustion-based heating elements may include, without limitation, catalytic combustion of materials such as methane, butane or propane gases. Physico/chemical heating elements include components undergoing phase change, such as in heat of condensation or crystallization. Chemical heating elements may include any combination of chemicals that when reacted release thermal energy. For example, H₂O₂ + MnO₂ (catalyst) causes an exothermic chemical reaction yielding H₂O + O₂. Other reactants would be apparent to those of ordinary skill. The thermal byproduct of such reactions, alone or in combination with another thermal supply, may provide sufficient energy to yield vaporization of the liquid dose, when contained in a properly insulated system.

It will be understood that various details of the invention may be changed without departing from the scope of the invention. Furthermore, the foregoing description is for the purpose of illustration only, and not for the purpose of limitation, as the invention is defined by the following, appended claims.

The application of which this description and claims form part may be used as a basis for priority in respect of any subsequent application. The claims of such subsequent application may be directed to any feature or combination of features described herein. They may take the form of product, composition, process or use claims and may include, by way of example and without limitation, one or more of the following claims.

## Claims

1. An inhaler apparatus comprising:
(a) a heating chamber reservoir (R) for containing a propellant that is liquid at room temperature and standard atmospheric pressure;
(b) a heating mechanism (HE), said heating mechanism being operable for heating said liquid propellant to at least a predetermined temperature threshold in a sealed manner to increase pressure inside said heating chamber reservoir;
(c) an actuator (P) for opening said apparatus and releasing for inhalation by a human said heated propellant; and
(d) a reservoir for containing said liquid propellant that is separate and distinct from said heating chamber reservoir,
**characterized by**
(e) in communication with said separate reservoir and with said heating chamber reservoir, a mechanism for metering said selected amount of said liquid propellant from said separate reservoir to said heating chamber reservoir,
wherein said heating chamber reservoir receives and heats a selected amount of said liquid propellant in a sealed manner to increase pressure within said heating chamber reservoir.

2. The inhaler apparatus of claim 1, further comprising said liquid propellant, and wherein said liquid propellant is a solvent having medicament dispersed therein, and said metering mechanism is for metering to said heating chamber reservoir said selected amount of solvent having dispersed therein a selected medicament dose.

3. The inhaler apparatus of claim 2, wherein said dose is in a form of being dispersed in solution in said solvent, or is in a form of being dispersed in suspension in said solvent.

4. The inhaler apparatus of claim 1, further comprising said heating chamber reservoir also is a reservoir for containing medicament dispersed in said liquid propellant.

5. The inhaler apparatus of claim 4, further comprising a metering mechanism in communication with said heating chamber reservoir for metering at least a selected amount of said medicament dispersed in said liquid propellant for delivery to a human.

6. The inhaler apparatus of claim 1, wherein said actuator is for releasing said heated propellant as a vapor.

7. The inhaler apparatus of claim 1, wherein said heating mechanism is operable for heating said liquid propellant to a temperature in a range from at least approximately 30°C above its boiling point in °C to at least approximately 80°C above its boiling point in °C.

8. The inhaler apparatus of claim 1, wherein said heating mechanism is operable for pressurizing said liquid propellant to a pressure increase greater than atmospheric pressure, when said liquid propellant is sealed inside said heating chamber reservoir.

9. The inhaler apparatus of claim 1, wherein said actuator for releasing said heated propellant is selected from the group consisting of a breath actuated actuator, a temperature actuated actuator, a pressure actuated actuator, and a manually actuated actuator.

10. The inhaler apparatus of claim 1, wherein said apparatus is a hand-held apparatus.

11. The inhaler apparatus of claim 2, wherein said propellant is selected from water, alkanols, glycols, ethanolamine, oleic acid, anti-oxidants, lecithin, and surfactants.

12. The inhaler apparatus of claim 4, wherein said heating chamber reservoir contains medicament dispersed in the liquid propellant.

13. The inhaler apparatus of claim 12, wherein said medicament is selected from the group consisting of fluticasone propionate and salbutamol sulfate.
